# EUROPÄISCHE PATENTSCHRIFT

(11) **EP 1 428 868 B1**
(45) Veröffentlichungstag und Bekanntmachung des Hinweises auf die Patenterteilung: **21.05.2014**
(21) Anmeldenummer: 03026831.2
(22) Anmeldetag: 20.11.2003
(51) Int. Cl.: C12M 1/107

(54) **Vorrichtung zur anaeroben Fermentation von Biomasse**
Device for anearobic fermentation of biomass
Dispositive pour fermentation anearobic de biomasse

(30) Priorität: 11.12.2002 DE 10257849
(43) Veröffentlichungstag der Anmeldung: 16.06.2004
(73) Patentinhaber: Schmack Biogas GmbH, 92421 Schwandorf (DE)
(72) Erfinder: Schiedermeier, Ludwig, 93449 Waldmunchen (DE)
(74) Vertreter: Möhring, Friedrich

(56) Entgegenhaltungen:
- DE-A- 3 715 952
- DE-A- 19 719 323
- DE-C- 3 134 980
- DE-C- 4 437 717
- US-A- 5 269 634

## Beschreibung

Die vorliegende Erfindung betrifft eine Vorrichtung zur anaeroben Fermentation von Biomasse.

Bei der anaeroben Fermentation (Vergärung) von biogenem Material handelt es sich um eine Alternative zu der bereits verbreitet eingesetzten Kompostierung. Dabei wird Biomasse in einem anaeroben Milieu von geeigneten Bakterien unter Bildung von Biogas (insbesondere Methan) zersetzt, wobei sowohl einstufige Verfahren wie auch zweistufige Verfahren (Hydrolysestufe und Methanisierungsstufe) vorgeschlagen wurden (vgl. z.B. DE 19516378 A1 und DE 19518234 A1).

Überwiegend ist dabei bisher an Verfahren der Naßfermentation gearbeitet worden, bei denen die Biomasse vor der Vergärung zur Erzeugung eines homogenen pump- und rührfähigen Breis aufbereitet wird (vgl. z.B. DE 3207048 A1). Nachteilig bei derartigen Verfahren ist der mit der Aufbereitung der Biomasse verbundene hohe Aufwand; zudem sind derartige Verfahren der Naßfermentation vergleichsweise störanfällig, insbesondere im Hinblick auf Fremdstoffe. Vor diesem Hintergrund ist in jüngster Zeit zunehmend die Trockenfermentation in das Interesse gerückt.

Aus der EP 0934998 A2 ist ein Verfahren zur Methanisierung von schüttfähigen, stapelbaren oder stückigmachbaren inoculierten Biomassen bekannt, bei dem der in einem Behälter angeordneten, von einer luftundurchlässigen Hülle (z.B. Folie) umgebenen Biomasse ein Impfmaterial als Einmalzugabe zugesetzt und die so gebildete Reaktionsmasse ohne weiteres Nachimpfen unter Luftabschluß vergoren wird. Zur Durchführung dieses Verfahrens wird durch die die Biomasse umgebende Hülle ein Einstechdorn in die Biomasse eingebracht, durch den hindurch die Biomasse zunächst zum Zwecke einer Vorrotte belüftet, anschließend mit einem flüssigen Impfmaterial geflutet und während der Vergärung das entstehende Biogas abgezogen wird. Alternativ kann das Impfmaterial bereits bei der Herstellung des mit Folie umwickelten Preßballens aus Biomasse beigemischt werden; in diesem Falle wird der Ballen nach der Vorrotte entweder aktiv oder passiv entlüftet, gegebenenfalls unter Zugabe von Wasser.

Die DE 4437717 C1 betrifft einen Hauptfermenter für ein- und mehrstufige Prozesse, der volumenveränderlich ausgebildet und mit einem Ausgleichsbehälter verbunden ist. Dieser Hauptfermenter soll alternativ zur Schaffung einer homogenen Biomasse und zur Trennung von Gasgemischen geeignet sein.

Die DE 3134980 offenbart eine Fermentationsvorrichtung mit einem beheizbaren Biogasreaktor, einem Biogas-Zwischenspeicher und einem Biogas-Hauptspeicher. Das in dem Biogasreaktor durch Vergärung der dort aufgenommenen Biomasse erzeugte, den Reaktor verlassende Biogas wird zunächst in dem Zwischenspeicher gesammelt. Durch Umschaltung entsprechender Ventile wird dann das in dem Zwischenspeicher gesammelte Biogas aus diesem über einen Düsenring in die in dem Reaktor aufgenommene Biomasse eingeblasen, wobei in dieser Prozeßphase das den Reaktor verlassende Biogas - zur weiteren Verwendung - in den Biogas-Hauptspeicher geleitet wird.

Weitere Biogasfermenter für verschiedene Biomasse-Ausgangsmaterialien unterschiedlicher Zusammensetzung und Konsistenz sind aus der EP 23176 A2, der WO 02/31104 A2, der FR 2498044, der FR 893537, der BE 470761 und der FR 905540 bekannt. Aufgrund ihrer spezifischen Konstruktionsmerkmale erscheinen diese Fermenter jeweils nur zur Verarbeitung bestimmter Ausgangsmaterialien mit spezifischen Eigenschaften innerhalb einer vergleichsweise engen Bandbreite als geeignet. Jedenfalls leidet bei diesen Fermentern die Effizienz erheblich, wenn das zu verarbeitende Ausgangsmaterial hinsichtlich seiner Zusammensetzung und seiner Konsistenz allzusehr von dem der Auslegung der jeweiligen Vorrichtung zugrundegelegten Material abweicht.

Im Lichte des vorstehend gewürdigten Standes der Technik liegt der vorliegenden Erfindung die Aufgabe zugrunde, eine Vorrichtung zur anaeroben Fermentation von Biomasse bereitzustellen, mit der sich stark unterschiedliche Ausgangsmaterialien mit einem hohen Wirkungsgrad verarbeiten lassen.

Gelöst wird diese Aufgabenstellung gemäß der vorliegenden Erfindung durch eine einen Fermenter, einen Biogas-Zwischenspeicher und einen Biogas-Hauptspeicher umfassende Vorrichtung zur anaeroben Fermentation von Biomasse, die sich durch die folgenden Merkmale auszeichnet:
- der Fermenter weist mindestens eine Fermentationszelle mit einem gasdurchlässigen Belüftungsboden auf;
- dem Biogas-Hauptspeicher (3) ist eingangsseitig ein absperrbares Steuerventil (20, 41) zugeordnet;
- der Biogas-Zwischenspeicher (2) und der Biogas-Hauptspeicher (3) sind ventilgesteuert über Biogasanschlüsse (16, 17) an unterschiedlichen Seiten des Belüftungsbodens (8) an die Fermentationszelle (5) anschließbar, wobei sich bei einer entsprechenden Druckdifferenz zwischen dem Biogas-Zwischenspeicher (2) und dem Biogas-Hauptspeicher (3) eine Ventilation von in dem Fermenter (1) enthaltener Biomasse (7) mit Biogas ergibt;
- es ist eine Heizeinrichtung (27) für die in dem Fermenter (1) enthaltene Biomasse (7) vorgesehen, welche einen in dem Zwischenspeicher (2) angeordneten Wärmetauscher (28) für das in ihm zwischengespeicherte Biogas umfasst.

Von besonderer Bedeutung für die erfindungsgemäße Vorrichtung ist somit, dass an den Fermenter zwei unterschiedliche Biogasspeicher angeschlossen sind, nämlich einerseits ein Zwischenspeicher und andererseits ein Hauptspeicher. Der Biogas-Hauptspeicher, aus dem das gewonnene Biogas zur weiteren Verwertung entnommen wird, ist dabei über ein zuströmseitig angeordnetes Steuerventil von dem Fermenter absperrbar. Ist in diesem Sinne der Biogas-Hauptspeicher von dem Fermenter abgekoppelt, so gelangt das durch die Vergärung der Biomasse entstehende Biogas in den Biogas-Zwischenspeicher. Durch geeignete Umsteuerung des Steuerventils strömt das in dem Zwischenspeicher zwischengespeicherte Biogas bei entsprechenden Druckverhältnissen aus diesem in den Biogas-Hauptspeicher, wobei es infolge der Anschließbarkeit der beiden Speicher an die mindestens eine Fermentationszelle auf unterschiedlichen Seiten des Belüftungsbodens die Biomasse in jener Fermentationszelle durchströmt. Hierdurch kommt es - allein aufgrund der Druckverhältnisse in den beiden Biogasspeichern, d.h. ohne Verwendung eines Energie verbrauchenden Gebläses - zu einer effizienten Ventila-an eine Verbrennungseinrichtung für das dem Hauptspeicher entnommene Biogas angeschlossen. Der genannte Wärmetauscher ist geeignet, das in dem Biogas-Zwischenspeicher zwischengespeicherte Biogas auf ein optimales Temperaturniveau zu erwärmen, bevor es in den Fermenter zurückgeleitet wird. Der hierdurch mögliche homogene und direkte Eintrag von Wärme in die zu vergärende Biomasse über das aufgeheizte, die Biomasse vom Zwischenspeicher zum Hauptspeicher durchströmende Biogas ist besonders effizient. Auf diese Weise läßt sich die Fermentation beschleunigen und somit die Verweildauer der Biomasse in dem Fermenter reduzieren, was sich ebenfalls positiv auf die Effizienz des Vergärungsverfahrens auswirkt.

Bevorzugt weist die Vorrichtung nach der vorliegenden Erfindung mindestens einen Kondensatabscheider auf, der dem den Fermenter verlassenden Biogas Feuchtigkeit entzieht. Indem auf diese Weise das zur Ventilation der Biomasse eingesetzte Biogas vergleichsweise trocken ist, kann es beim Durchströmen der Biomasse erneut Feuchtigkeit aufnehmen. Das Resultat ist ein am Ende der Vergärung vorliegendes Substrat mit einem besonders hohen Gehalt an Trockensubstanz.

Gemäß einer anderen bevorzugten Weiterbildung der Erfindung ist der Biogas-Zwischenspeicher als Konstantdruckspeicher ausgeführt, dessen Druck unabhängig ist von dem jeweiligen Füllzustand. Auf diese Weise lassen sich die innerhalb des Fermenters bestehenden Druckverhältnisse in jenen Phasen, in denen - bei gesperrter Zufuhr des Biogas-Hauptspeichers - das durch die Vergärung entstehende Biogas in den Biogas-Zwischenspeicher geleitet wird, vergleichmäßigen, was der Biogas-Produktion durch die - z.T. auf Druckschwankungen empfindlichen anaeroben Mikroorganismen - entgegenkommt. Gegebenenfalls kann dabei dem Biomasse-Zwischenspeicher eine Druckerhöhungseinrichtung zugeordnet sein, mittels welcher sich der Druck innerhalb des Biogas-Zwischenspeichers in jenen Betriebsphasen erhöhen läßt, in denen Biogas zur Ventilation der Biomasse von dem Biogas-Zwischenspeicher in den Biogas-Hauptspeicher strömt. Eine solche Druckerhöhungseinrichtung kann vergleichsweise einfach ausgeführt sein, beispielsweise in Form einer Zusatzlast, mit welcher ein kolbenförmiges Bauteil des Zwischenspeichers in den genannten Betriebsphasen belastet wird.

Eine andere bevorzugte Weiterbildung der erfindungsgemäßen Vorrichtung zeichnet sich dadurch aus, daß der Biogas-Zwischenspeicher über ventilgesteuerte Biogasanschlüsse an die Fermentationszelle wahlweise an jede der beiden Seiten des Belüftungsbodens anschließbar ist. Eine solche Gestaltung erweist sich insbesondere dann als günstig, wenn der Fermenter lediglich eine einzige Fermentationszelle aufweist, namentlich wenn er als garagenförmiger Fermenter entsprechend der WO 02/31104 ausgeführt ist. Sowohl in jener Betriebsphase, in der das bei der Vergärung entstehende Biogas in den Zwischenspeicher gelangt, wie auch während der Ventilationsphase, in der das Biogas dem Hauptspeicher zugeführt wird, kann das Biogas die Fermentationszelle in dem Bereich oberhalb der Biomasse verlassen, wobei das ventilierte Biogas die Biomasse von unten nach oben durchströmt. Dies ist sowohl unter strömungstechnischen Gesichtspunkten als auch im Hinblick auf eine möglichst geringe Anwesenheit von Fremdstoffen in dem Biogas besonders günstig.

Gemäß einer anderen bevorzugten Weiterbildung der Erfindung ist der Fermenter siloartig ausgeführt und weist mehrere übereinander angeordnete Fermentationszellen auf, wobei die Belüftungsböden jeweils durch einen gasdurchlässigen Klappboden gebildet sind und der Biogas-Zwischenspeicher und der Biogas-Hauptspeicher über Biogasanschlüsse alternierend an die einzelnen Fermentationszellen anschließbar sind. Der Fermenter kann dabei in seinem oberen Bereich eine Aufgabeschleuse und in seinem unteren Bereich eine Abzugschleuse aufweisen. Bei Anwendung dieses Fermenmters läßt sich die Verfahrensführung bei einem vergleichsweise geringen technischen Aufwand einem quasi-kontinuierlichen Betrieb annähern. In vergleichsweise kurzen Abständen (z.B. alle zwei Tage) gelangt die teilweise vergorene Biomasse durch Öffnen des Klappbodens der betreffenden Fermentationszelle in die darunterliegende Fermentationszelle. Dabei wird das Material aufgelockert und durchmischt, so daß sich bei der Fortsetzung des Vergärungsverfahrens in der nächsten Fermentationszelle aufgrund der gesteigerten Homogenität der Biomasse eine entsprechend hohe Effizienz mit guter Gasausbeute ergibt.

Besonders günstig ist es dabei, wenn der obersten Fermentationszelle ein Perkolatverteiler und der untersten Fermentationszelle ein Perkolatsammler, die an eine Perkolatzirkulation angeschlossen sein können, zugeordnet sind. In diesem Falle läßt sich das Milieu, in welchem sich die Vergärung vollzieht, besonders effizient optimieren.

Eine andere bevorzugte Weiterbildung der Erfindung zeichnet sich dadurch aus, daß der Fermenter Teil eines kombinierten Anaerob-aerob-Reaktors ist, der mindestens eine dem Fermenter vor- oder nachgeschaltete, belüftete Rottezelle aufweist, die von der angrenzende Fermentationszelle über einen im wesentlichen luftdichten Klappboden getrennt ist. In diesem Falle kann insbesondere das sich bei der Vergärung ergebende Substrat ohne nennenswerten Aufwand einer anschließenden aeroben Fermentation (Rotte) unterzogen werden, indem es durch Öffnen des Klappbodens der untersten Fermentationszelle des Fermenters in die darunter angeordnete Rottezelle gelangt. Die luftdichte Ausführung des die Rottezelle von der darüberliegenden Fermentationszelle trennenden Klappbodens stellt dabei sicher, daß sich im Betrieb des Anaerob-aerob-Reaktors in dem Fermenter ein anaerobes Verfahren und in der Rottezelle zugleich ein aerobes Verfahren betreiben läßt. Gegebenenfalls kann dabei vorgesehen sein, daß bei geöffnetem Klappboden der untersten Fermentationszelle in der Rottezelle über ein entsprechendes Sauggebläse ein Unterdruck erzeugt wird, so daß kurzzeitig Biogas aus dem Zwischenspeicher über die unterste Fermentationszelle in die Rottezelle gesaugt wird, um ein Eindringen von Luft in letztere zu verhindern.

Im folgenden wird die vorliegende Erfindung anhand zweier in der Zeichnung veranschaulichter bevorzugter Ausführungsbeispiele näher erläutert. Dabei zeigt
- Fig. 1: eine einen garagenförmigen Fermenter umfassende Vorrichtung und
- Fig. 2: eine einen Anaerob-aerob-Reaktor mit einem siloartigen Fermenter umfassende Vorrichtung nach der vorliegenden Erfindung.

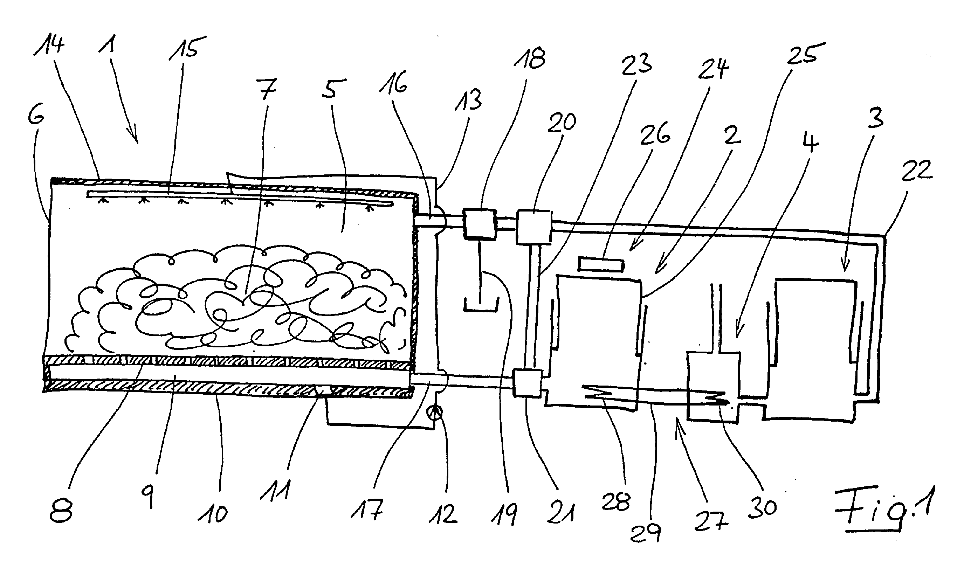

Die in Fig. 1 schematisch veranschaulichte Vorrichtung zur anaeroben Fermentation von Biomasse umfaßt einen Fermenter 1, einen Biogas-Zwischenspeicher 2, einen Biogas-Hauptspeicher 3 sowie ein Blockheizkraftwerk 4. Der Fermenter 1 ist garagenförmig ausgeführt; er umfaßt eine einzige Fermentationszelle 5, die durch ein Tor 6 mit zu vergärender Biomasse 7 beschickbar und entleerbar ist.

Die Biomasse 7 ruht auf einem Belüftungsboden 8, unter dem eine Belüftungskammer 9 angeordnet ist. In dem Boden 10 der Belüftungskammer 9 ist ein Perkolatsammler 11 vorgesehen, welcher an eine eine Umwälzpumpe 12 umfassende Perkolatzirkulation 13 angeschlossen ist, mittels welcher das in dem Perkolatsammler 11 aufgefangene Perkolat einem unterhalb der Decke 14 des Fermenters 1 angeordneten Perkolat-Verteiler 15 zugeführt wird.

In die Fermentationszelle 5 münden zwei Biogasanschlüsse 16 und 17, nämlich ein erster Biogasanschluß 16 oberhalb des Belüftungsbodens 8 und ein zweiter Biogasanschluß 17 unterhalb des Belüftungsbodens 8. Der erste Biogasanschluß 16 ist dabei im oberen Randbereich an die Fermentationszelle 5 angeschlossen, während der zweite Biogasanschluß 17 an die Belüftungskammer 9 angeschlossen ist. Im Bereich des ersten Biogasanschlusses 16 ist ein Kondensat-Abscheider 18 vorgesehen. Die Flüssigkeit, die dem durch den ersten Biogasanschluß 16 strömenden Biogas durch den Kondensat-Abscheider 18 entzogen wird, kann je nach der Ausführung der Anlage entsorgt oder aber weiterverwertet werden.

Dem ersten Biogasanschluß 16 ist ein Dreiwegeventil 20 und dem zweiten Biogasanschluß 17 ist ein Dreiwegeventil 21 zugeordnet. Mittels des Dreiwegeventils 20 kann dabei der erste Biogasanschluß 16 entweder mit der Einspeisleitung 22, welche zuströmseitig an den Biogas-Hauptspeicher 3 angeschossen ist, oder aber mit der Verbindungsleitung 23, welche die beiden Dreiwegeventile 20 und 21 miteinander verbindet, verbunden werden. Mittels des Dreiwegeventils 21 kann der Biogas-Zwischenspeicher 2 entweder an dem zweiten Biogasanschluß 17 oder aber an die Verbindungsleitung 23 angeschossen werden.

Der Biogas-Zwischenspeicher 2 ist als Konstantdruckspeicher ausgeführt. Der in ihm herrschende Druck ist somit unabhängig von seinem Füllgrad, es sei denn, es wird eine Druckerhöhungseinrichtung 24 aktiviert, welche im einfachsten Fall eine auf das Kolbenelement 25 des Zwischenspeichers 2 absenkbare Zusatzlast 26 umfaßt.

Ausgangsseitig ist der Biogas-Hauptspeicher 3 an das Blockheizkraftwerk 4 angeschlossen. Ein Teil der in dem Blockheizkraftwerk 4 bei der Verbrennung des dem Biogas-Hauptspeicher 3 entnommenen Biogases entstehenden Wärme wird über eine Heizeinrichtung 27 dem in dem Zwischenspeicher 2 zwischengespeicherten Biogas zugeführt. Hierzu ist im Inneren des Biogas-Zwischenspeichers 2 ein Wärmetauscher 28 angeordnet, welcher über entsprechende Leitungen 29 mit einem dem Blockheizkraftwerk 4 zugeordneten Wärmetauscher 30 in Verbindung steht.

Die beiden Dreiwegeventile 20 und 21 werden über eine entsprechende Steuerung abwechselnd so eingestellt, daß während einer ersten Betriebsphase des Fermenters 1 das in diesem gebildete Biogas den Fermenter über den Biogasanschluß 16 verläßt und über das Dreiwegeventil 20, die Zwischenleitung 23 und das Dreiwegeventil 21 in den Biogas-Zwischenspeicher 2 gelangt. Der Biogas-Hauptspeicher 3 ist während dieser Betriebsphase durch entsprechende Stellung des Dreiwegeventils 20 von dem Fermenter 1 getrennt. In einer zweiten Betriebsphase ist demgegenüber durch entsprechende Steuerung der beiden Dreiwegeventile 20 und 21 der Biogasanschluß 17 mit dem Biogas-Zwischenspeicher 2 und der Biogasanschluß 16 mit dem Biogas-Hauptspeicher 3 verbunden. Bei einer entsprechenden Druckdifferenz zwischen dem Biogas-Zwischenspeicher 2 und dem Biogas-Hauptspeicher 3 ergibt sich eine Ventilation der Biomasse 7 mit Biogas, welches - durch die Heizeinrichtung 27 aufgeheizt - die Biomasse auf dem Weg vom Zwischenspeicher in den Hauptspeicher von unten nach oben durchströmt. Die Druckverhältnisse lassen sich unter Verwendung der Druckerhöhungseinrichtung 24 im Hinblick auf eine optimale Ventilation beeinflussen.

Ersichtlich kann die in der Zeichnung veranschaulichte Gestaltung der Biogasleitungen und der Steuerventile in erheblichem Umfang modifiziert werden, ohne daß dies unmittelbare Auswirkungen auf die beschriebene Betriebsweise der Vorrichtung als solche hätte. Beispielsweise ist bei Bedarf die Integration von Rückschlagventilen möglich; und der Biogas-Hauptspeicher kann über eine eigene, ventilgesteuerte Biogasleitung direkt an den Fermenter angeschlossen sein. In entsprechender Anwendung der veranschaulichten Komponenten ist ferner möglich, mehrere Fermenter miteinander vernetzt an einen gemeinsamen Biogas-Zwischenspeicher und einen gemeinsamen Biogas-Hauptspeicher anzuschließen.

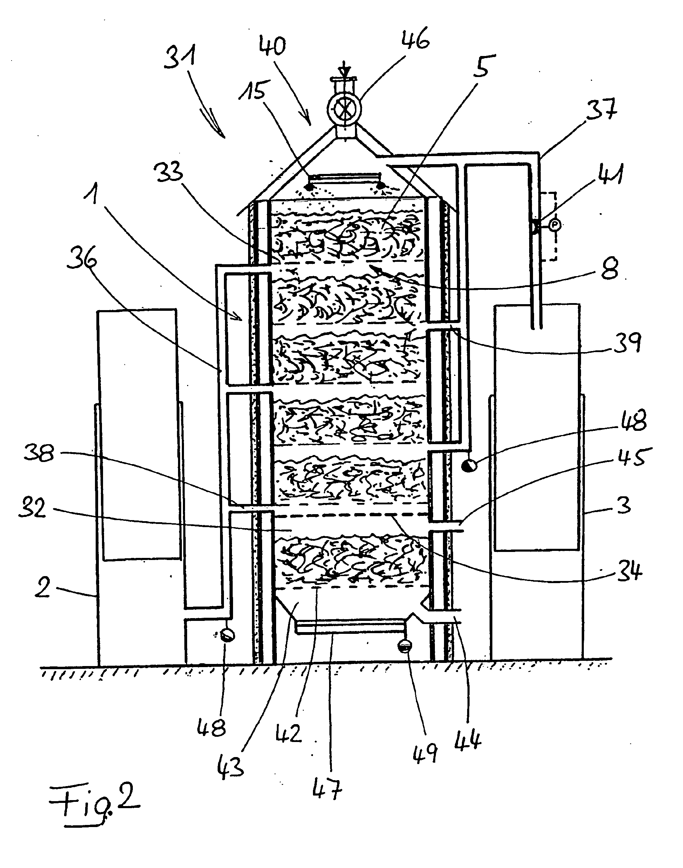

Bei der in Fig. 2 veranschaulichten Vorrichtung ist der Fermenter 1 Teil eines Anaerob-aerob-Reaktors 31, welcher fünf Fermentationszellen 5 und eine Rottezelle 32 umfaßt. Die fünf Fermentationszellen 5 sind dabei etagenweise übereinander angeordnet. Ihre Belüftungsböden 8 sind jeweils als Klappboden 33 ausgeführt. Durch Öffnen des Klappbodens 33 einer Fermentationszelle gelangt die Biomasse 7 aus dieser Fermentationszelle in die nächst tiefer gelegene. Die Rottezelle 32 ist unterhalb der untersten der fünf Fermentationszellen angeordnet, gegenüber der sie durch einen luftdicht verschließbaren Klappboden 34 im normalen Betrieb des Reaktors 31 hermetisch abgeschlossen ist. Im Bereich seiner Außenwand weist der Reaktor 31 eine Wärmeisolierung 35 auf.

Der Biogas-Zwischenspeicher 2 und der Biogas-Hauptspeicher 3 sind über zugeordnete Biogasleitungen 36 bzw. 37 alternierend an die Fermentationszellen 5 angeschlossen, indem die dem Biogas-Zwischenspeicher 2 zugeordnete Biogasleitung 36 über entsprechende Zweigleitungen 38 unterhalb des Belüftungsbodens der untersten, der mittleren und der obersten Fermentationszelle und die dem Biogas-Hauptspeicher 3 zugeordnete Biogasleitung 37 über entsprechende Zweigleitungen 39 unterhalb der Belüftungsbodens der zweiten und vierten Fermentationszelle sowie im Bereich der den Reaktor 31 nach oben abschließenden Kuppel 40 mündet. Dieser Anschluß der fünf Fermentationszellen an den Biogas-Zwischenspeicher 2 und den Biogas-Hauptspeicher 3 ermöglicht entsprechend den weiter oben dargelegten Erläuterungen die Ventilation der fünf Fermentationszellen mit Biogas, welches aufgrund einer entsprechenden Druckdifferenz vom Biogas-Zwischenspeicher in den Biogas-Hauptspeicher gelangt, wobei bei der entsprechenden Ventilation die unterste, die mittlere und die oberste Fermentationszelle von unten nach oben mit Biogas durchströmt werden und die zweite und die vierte Fermentationszelle von oben nach unten. In den Zweigleitungen 38 und 39 können Ventile vorgesehen sein, mittels derer sich die Strömungsverhältnisse so einstellen lassen, daß die Ventilation der einzelnen Fermentationszellen optimiert ist.

In der Biogasleitung 37 ist im Bereich der Zuströmseite des Biogas-Hauptspeichers 3 ein Steuerventil 41 angeordnet, mittels dessen sich der Biogas-Hauptspeicher von dem Fermenter 1 abtrennen läßt, so daß in der entsprechenden Betriebsphase des Fermenters sämtliches produzierte Biogas in den Biogas-Zwischenspeicher 2 gelangt.

Was die Rottezelle 32 angeht, so ist diese zwangsbelüftet. In die unterhalb des Belüftungsbodens 42 angeordnete Belüftungskammer 43 mündet ein Frischluftstutzen 44, und unterhalb des luftdicht verschließbaren Klappbodens 34 mündet ein Abluftstutzen 45 in die Rottezelle 32. Der Abluftstutzen 45 ist an ein - nicht dargestelltes - Sauggebläse angeschossen. Der hierdurch im Bereich der Rottezelle 32 erzeugte Unterdruck verhindert, daß bei geöffnetem Klappboden 34 Luft in den an-aerob arbeitenden Fermenter 1 eindringt.

Im Bereich der Kuppel 40 weist der Reaktor 31 eine - als solches bekannte - Biomasse-Aufgabeschleuse 46 auf. Unterhalb der Rottezelle 32 ist eine Abzugschleuse 47 für das den Reaktor verlassende Substrat vorgesehen.

Im Bereich der Gasleitungen 36 und 37 sind Kondensatabscheider 48 und im Bereich der Kuppel 40 ist eine Perkolatzufuhr 15 vorgesehen. In Anbetracht der Beschreibung zu Fig. 1 bedarf es für diese Komponenten keiner weiteren Erläuterungen. Entsprechendes gilt für eine - nicht dargestellte - Verwertung des dem Biogas-Hauptspeicher entnommenen Biogases sowie eine - ebenfalls nicht dargestellte - Heizeinrichtung, mittels welcher das in dem Biogas-Zwischenspeicher zwischengespeicherte Biogas aufgeheizt werden kann. Ein - nicht dargestellter - Perkolatsammler ist zweckmäßigerweise im Bereich des den Fermenter nach unten begrenzenden Klappbodens 34 vorgesehen. Im Bereich der Abzugschleuse 47 kann mittels eines Abscheiders 49 bei der Rotte freigesetzte Flüssigkeit aufgefangen und einer Aufbereitung zugeführt werden.

## Patentansprüche

1. Vorrichtung zur anaeroben Fermentation von Biomasse (7), umfassend einen Fermenter (1), einen Biogas-Zwischenspeicher (2) und einen Biogas-Hauptspeicher (3), mit den folgenden Merkmalen:
- der Fermenter (1) weist mindestens eine Fermentationszelle (5) mit einem gasdurchlässigen Belüftungsboden (8) auf;
- dem Biogas-Hauptspeicher (3) ist eingangsseitig ein absperrbares Steuerventil (20, 41) zugeordnet;
- der Biogas-Zwischenspeicher (2) und der Biogas-Hauptspeicher (3) sind ventilgesteuert über Biogasanschlüsse (16, 17) an unterschiedlichen Seiten des Belüftungsbodens (8) an die Fermentationszelle (5) anschließbar, wobei sich bei einer entsprechenden Druckdifferenz zwischen dem Biogas-Zwischenspeicher (2) und dem Biogas-Hauptspeicher (3) eine Ventilation von in dem Fermenter (1) enthaltener Biomasse (7) mit Biogas ergibt;
- es ist eine Heizeinrichtung (27) für die in dem Fermenter (1) enthaltene Biomasse (7) vorgesehen, welche einen in dem Zwischenspeicher (2) angeordneten Wärmetauscher (28) für das in ihm zwischengespeicherte Biogas umfasst.

2. Vorrichtung nach Anspruch 1,
**dadurch gekennzeichnet,**
**daß** der Wärmetauscher (28) an eine Verbrennungseinrichtung für das dem Hauptspeicher (3) entnommene Biogas angeschlossen ist.

3. Vorrichtung nach einem der Ansprüche 1 oder 2,
**dadurch gekennzeichnet,**
**daß** der Biogas-Zwischenspeicher (2) als Konstantdruckspeicher ausgeführt ist.

4. Vorrichtung nach einem der Ansprüche 1 bis 3,
**dadurch gekennzeichnet,**
**daß** dem Biomasse-Zwischenspeicher (2) eine Druckerhöhungseinrichtung (24) zugeordnet ist.

5. Vorrichtung nach einem der Ansprüche 1 bis 4,
**dadurch gekennzeichnet,**
**daß** der Biogas-Zwischenspeicher (2) über ventilgesteuerte Biogasanschlüsse (16, 17) an die Fermentationszelle (5) wahlweise an jede der beiden Seiten des Belüftungsbodens (8) anschließbar ist.

6. Vorrichtung nach einem der Ansprüche 1 bis 5,
**gekennzeichnet durch** die folgenden Merkmale:
- der Fermenter (1) ist siloartig ausgeführt und weist mehrere übereinander angeordnete Fermentationszellen (5) auf, wobei die Belüftungsböden (8) jeweils **durch** einen gasdurchlässigen Klappboden (33) gebildet sind;
- der Biogas-Zwischenspeicher (2) und der Biogas-Hauptspeicher (3) sind über Biogasanschlüsse (36, 38; 37, 39) alternierend an die einzelnen Fermentationszellen (5) anschließbar.

7. Vorrichtung nach Anspruch 6,
**dadurch gekennzeichnet,**
**daß** der Fermenter (1) in seinem oberen Bereich eine Aufgabeschleuse (46) und in seinem unteren Bereich eine Abzugschleuse (47) aufweist.

8. Vorrichtung nach Anspruch 6 oder Anspruch 7,
**dadurch gekennzeichnet,**
**daß** der obersten Fermentationszelle (5) ein Perkolatverteiler (15) zugeordnet ist.

9. Vorrichtung nach einem der Ansprüche 6 bis 8,
**dadurch gekennzeichnet,**
**daß** der untersten Fermentationszelle (5) ein Perkolatsammler zugeordnet ist.

10. Vorrichtung nach einem der Ansprüche 6 bis 9,
**dadurch gekennzeichnet,**
**daß** der Fermenter (1) Teil eines kombinierten Anaerob-aerob-Reaktors (31) ist, der mindestens eine dem Fermenter vor- oder nachgeschaltete, belüftete Rottezelle (32) aufweist, die von der angrenzenden Fermentationszelle (5) über einen im wesentlichen luftdichten Klappboden (34) getrennt ist.

11. Vorrichtung nach einem der Ansprüche 1 bis 10,
**dadurch gekennzeichnet,**
**daß** mindestens einem der Biogasanschlüsse (16, 17) ein Kondensatabscheider (18) zugeordnet ist.

## Claims

1. A device for the anaerobic fermentation of biomass (7), comprising a fermenter (1), a temporary biogas storage tank (2) and a main biogas storage tank (3), having the following features:
- the fermenter (1) has at least one fermentation cell (5) with a gas-permeable aeration floor (8);
- an isolatable control valve (20, 41) is assigned to the main biogas storage tank (3) on the inlet side;
- the temporary biogas storage tank (2) and the main biogas storage tank (3) are connectable in a valve-controlled manner via biogas connections (16, 17) at different ends of the aeration floor (8) to the fermentation cell (5), wherein with a corresponding pressure difference between the temporary biogas storage tank (2) and the main biogas storage tank (3), ventilation of biomass (7) contained in the fermenter (1) with biogas results;
- a heating device (27) is provided for the biomass (7) contained in the fermenter (1), which heating device comprises a heat exchanger (28) disposed in the temporary storage tank for the biogas temporarily stored therein.

2. The device according to claim 1,
**characterized in that**
the heat exchanger (28) is connected to a combustion device for the biogas removed from the main storage tank (3).

3. The device according to one of the claims 1 or 2,
**characterized in that**
the temporary biogas storage tank (2) is designed as a constant pressure accumulator.

4. The device according to one of the claims 1 to 3,
**characterized in that**
a pressure-increasing mechanism (24) is assigned to the temporary biomass storage tank (2).

5. The device according to one of the claims 1 to 4,
**characterized in that**
the temporary biogas storage tank (2) is optionally connectable via valve-controlled biogas connections (16, 17) to the fermentation cell (5) at each of the two sides of the aeration floor (8).

6. The device according to one of the claims 1 to 5, **characterized by** the following features:
- the fermenter (1) is designed in the manner of a silo and has a plurality of fermentation cells (5) arranged above one another, wherein the aeration floors (8) are each formed by a gas-permeable hinged floor (33);
- the temporary biogas storage tank (2) and the main biogas storage tank (3) are alternately connectable to the individual fermentation cells (5) via biogas connections (36, 38, 37, 39).

7. The device according to claim 6,
**characterized in that**
the fermenter (1) has a discharge lock (46) in its upper region and an extraction lock (47) in its lower region.

8. The device according to claim 6 or claim 7,
**characterized in that**
the uppermost fermentation cell (5) is assigned a percolate distributor (15).

9. The device according to one of the claims 6 to 8,
**characterized in that**
the lowermost fermentation cell (5) is assigned a percolate collector.

10. The device according to one of the claims 6 to 9,
**characterized in that**
the fermenter (1) is part of a combined anaerobic/ aerobic reactor (31) which has at least one aerated rotting compartment (32) upstream or downstream of the fermenter, which rotting compartment is separated from the adjacent fermentation cell (5) via a substantially airtight hinged floor (34).

11. The device according to one of the claims 1 to 10,
**characterized in that**
at least one of the biogas connections (16, 17) is assigned a condensate separator (18).

## Revendications

1. Dispositif pour la fermentation anaérobie d'une biomasse (7) comprenant un fermenteur (1), un accumulateur intermédiaire (2) de biogaz et un accumulateur principal (3) de biogaz, avec les attributs suivants :
- le fermenteur (1) comporte au moins une cellule de fermentation (5) avec un fond d'aération (8) perméable au gaz ;
- à l'accumulateur principal (3) de biogaz est associée du côté entrée une soupape de commande (20, 41) obturable ;
- l'accumulateur intermédiaire (2) de biogaz et l'accumulateur principal (3) de biogaz peuvent se raccorder en étant commandés par soupape via des raccords (16, 17) de biogaz par différents côtés du fond d'aération (8) sur la cellule de fermentation (5), sachant que lors d'une pression différentielle correspondante entre l'accumulateur intermédiaire (2) de biogaz et l'accumulateur principal (3) de biogaz, il s'effectue une ventilation de la biomasse (7) contenue dans le fermenteur (1) avec du biogaz ;
- il est prévu pour la biomasse (7) contenue dans le fermenteur (1) un système de chauffage (27) qui comprend un échangeur thermique (28) placé dans l'accumulateur intermédiaire (2) pour le biogaz qui y est temporairement accumulé.

2. Dispositif selon la revendication 1,
**caractérisé en ce que**
l'échangeur thermique (28) est raccordé sur un système de combustion pour le biogaz prélevé dans l'accumulateur principal (3).

3. Dispositif selon l'une quelconque des revendications 1 ou 2,
**caractérisé en ce que**
l'accumulateur intermédiaire (2) de biogaz est réalisé en tant qu'accumulateur constant de pression.

4. Dispositif selon l'une quelconque des revendications 1 à 3,
**caractérisé en ce qu'**
à l'accumulateur intermédiaire (2) de biomasse est associé un système d'augmentation de pression (24).

5. Dispositif selon l'une quelconque des revendications 1 à 4,
**caractérisé en ce que**
l'accumulateur intermédiaire (2) de biogaz peut se raccorder sur la cellule de fermentation (5) via des raccords de biogaz (16, 17) commandés par soupape, au choix sur chacun des deux côtés des fonds d'aération (8) .

6. Dispositif selon l'une quelconque des revendications 1 à 5,
**caractérisé par** les attributs suivants :
- le fermenteur (1) est réalisé à la manière d'un silo et comporte plusieurs cellules de fermentation (5) superposées, les fonds d'aération (8) étant formés chacun par un fond rabattable (33) perméable au gaz ;
- l'accumulateur intermédiaire (2) de biogaz et l'accumulateur principal (3) de biogaz peuvent se raccorder en alternance via des raccords de biogaz (36, 38 ; 37, 39) sur chacune des cellules de fermentation (5).

7. Dispositif selon la revendication 6,
**caractérisé en ce que**
le fermenteur (1) comporte dans sa zone supérieure un sas d'alimentation (46) et dans sa zone inférieure un sas de soutirage (47).

8. Dispositif selon la revendication 6 ou la revendication 7,
**caractérisé en ce qu'**
à la cellule de fermentation (5) supérieure est associé un répartiteur de percolats (15).

9. Dispositif selon l'une quelconque des revendications 6 à 8,
**caractérisé en ce qu'**
à la cellule de fermentation (5) inférieure est associé un collecteur de percolats.

10. Dispositif selon l'une quelconque des revendications 6 à 9,
**caractérisé en ce que**
le fermenteur (1) est une partie d'un réacteur anaérobie/aérobie (31) mixte qui comporte au moins une cellule de rouissage (32) aérée montée en avant ou en aval du fermenteur, qui est séparée de la cellule de fermentation (5) adjacente via un fond rabattable (34) sensiblement étanche à l'air.

11. Dispositif selon l'une quelconque des revendications 1 à 10,
**caractérisé en ce qu'**
à au moins l'un des raccords de biogaz (16, 17) est associé un séparateur de condensats (18).
